**Europäisches Patentamt**

**European Patent Office**

**Office européen des brevets**

(19)

(11) Veröffentlichungsnummer : **0 338 979 B1**

(12)

# EUROPÄISCHE PATENTSCHRIFT

(45) Veröffentlichungstag der Patentschrift :
05.08.92 Patentblatt 92/32

(51) Int. Cl.⁵ : **A61M 29/02,** A61M 25/10,
A61B 17/02

(21) Anmeldenummer : **89810248.8**

(22) Anmeldetag : **04.04.89**

(54) **Katheter zum Rekanalisieren von verengten Gefässen.**

(30) Priorität : **20.04.88 CH 1460/88**

(43) Veröffentlichungstag der Anmeldung :
**25.10.89 Patentblatt 89/43**

(45) Bekanntmachung des Hinweises auf die
Patenterteilung :
**05.08.92 Patentblatt 92/32**

(84) Benannte Vertragsstaaten :
**AT BE CH DE ES FR GB GR IT LI LU NL SE**

(56) Entgegenhaltungen :
**CH-A- 654 214
DE-A- 2 805 269
DE-A- 3 442 736**

(73) Patentinhaber : **SCHNEIDER (EUROPE) AG
Ackerstrasse 6
CH-8180 Bülach (CH)**

(72) Erfinder : **Schneider, Ernst, Dr.
Bolleystrasse 45
CH-8006 Zürich (CH)**

(74) Vertreter : **Groner, Manfred et al
Patentanwalts-Bureau Isler AG Postfach 6940
CH-8023 Zürich (CH)**

## Beschreibung

Die Erfindung betrifft einen Katheter nach dem Oberbegriff des Anspruchs 1.

Katheter dieser Art sind bekannt siehe z.B. DE-A-3 442 736 und werden insbesondere zur Behandlung von Gefässverengungen seit einiger Zeit eingesetzt. Diese Katheter besitzen am vorderen Ende einen unter fluidem Druck aufblähbaren Ballon, der in gefaltetem Zustand mit Hilfe eines Führungsdrahtes in den Bereich der Engstelle gebracht wird. Durch einen von aussen zugänglichen und durch den Trägerschlauch führenden Kanal wird der Ballon mit Flüssigkeit gefüllt, so dass dieser sich entfaltet und das verengende Gewebe nach aussen an oder in die Gefässwand drückt.

Nachteilig ist, dass die Behandlung bei starken Verengungen vergleichsweise lange dauert und deshalb zur Vermeidung beispielsweise einer distalen Ischämie die Behandlung kurzzeitig unterbrochen werden muss. Es hat sich auch gezeigt, dass ein langfristiger Erfolg der Behandlung oft nicht erreicht wird, da das verengende Gewebe letztlich nicht entfernt, sondern lediglich an die Gefässwand gepresst wird. Der Erfolg einer solchen Behandlung lässt sich verbessern, wenn in das zu behandelnde Gefäss beispielsweise eine das störende Gewebe auflösende Substanz, beispielsweise ein Plasminogen-Aktivator eingebracht wird, wozu allerdings vergleichsweise viel Substanz benötigt wird.

Der Erfindung liegt die Aufgabe zugrunde, einen Katheter der eingangs genannten Art zu schaffen, mit dessen Hilfe insbesondere Gefässverengungen mit besserem langzeitigen Erfolg behandelt werden können und der dennoch einen einfachen Aufbau aufweist und bei kostengünstiger Herstellung zuverlässig ist.

Die Aufgabe wird durch die Erfindung gemäss Anspruch 1 gelöst.

Die Flüssigkeit, welche in den Innenraum des Dilatationselements gepumpt wird, dringt bei genügend Druck durch die mikroporöse Wandung direkt in das anliegende Gewebe. Indem nun der Flüssigkeit Substanzen beigemischt bzw. in dieser gelöst werden, können diese direkt in das zu beseitigende Gewebe eingebracht werden. Werden Substanzen eingesetzt, welche das Gewebe auflösen, so lässt sich einerseits die Behandlungszeit wesentlich verkürzen und anderseits der langfristige Erfolg der Behandlung verbessern, da das störende Gewebe nicht nur verdrängt sondern aus dem Gefäss entfernt wird. Sehr wesentlich ist, dass die Substanz ohne Verlust direkt in das zu entfernende Gewebe eingebracht wird und somit lediglich die minimale Menge der Substanz benötigt wird. Weil die Substanz direkt an den Wirkungsort gebracht wird, wirkt sie schnell und gezielt. Vorteilhafte Ausgestaltungen der Erfindung sind in den abhängigen Ansprüchen gekennzeichnet.

Ein Ausführungsbeispiel der Erfindung wird nun anhand der Zeichnung näher erläutert. Es zeigen:

Fig. 1 im Längsschnitt einen erfindungsgemässen Katheter im ausgedehnten Zustand.

Fig. 2 im Längsschnitt ein Blutgefäss mit einem eingeführten Katheter,

Fig. 3 einen Querschnitt entlang der Linie III-III in Fig. 2.

Der Katheter weist an seinem vorderen Ende einen Dilatationsballon 1 auf, der über ein Oeffnung 9 eines Verbindungsstückes 6 mit einem Kanal 4 eines zweilumigen Trägerschlauchs 3 verbunden ist. Mit Hilfe einer hier nicht gezeigten, am anderen Ende des Trägerschlauchs 3 angeordneten Saug-Pumpe lässt sich der Dilatationsballon 1 für seine Einführung beispielsweise in ein Blutgefäss falten. Vor der Einführung des Dilatationsballons 1 wird zunächst beispielsweise ein stenosierter Gefässabschnitt mit einem bekannten Führungsdraht 2 sondiert und dann der Dilatationsballon 1 im gefalteten Zustand auf dem Führungsdraht 2 an den zu dilatierenden Gefässabschnitt geschoben. Zur Lokalisierung des Dilatationsballons 1 sind auf einem Schlauchstück 7 röntgenographisch gut sichtbare Markierelemente 8 angebracht.

Ist der gefaltete Dilatationsballon 1 im vorgesehenen Gefässabschnitt, so wird er mit Hilfe einer hier nicht gezeigten Druck-Pumpe unter kontrolliertem Druck mit einer Flüssigkeit 13 gefüllt, bis er die in der Zeichnung gezeigte Form annimmt. Der Dilatationsballon 1 ist so ausgeführt, dass er nach dem Erreichen einer bestimmten Form auch dann keinen grösseren Durchmesser annimmt, wenn ein erheblich höherer Druck ausgeübt wird. Damit wird vermieden, dass das Gefäss ungewollt zu stark gedehnt und beschädigt wird.

Der beispielsweise aus Polyvinylchlorid bestehende Dilatationsballon 1 weist eine im dilatierten Zustand zylindrisch Wandung 5 mit mehreren Durchstichen 12 auf. Die Durchstiche 12 sind so dimensioniert und angeordnet, dass im dilatierten Zustand die Flüssigkeit 13 durch die Wandung 5 hindurch nach aussen dringt. Die Durchstiche 12 besitzen einen vergleichsweise kleinen Durchmesser von beispielsweise 0,05 mm, so dass also die Wandung 5 mikroporös, aber für die Flüssigkeit 13 dennoch permeabel ist.

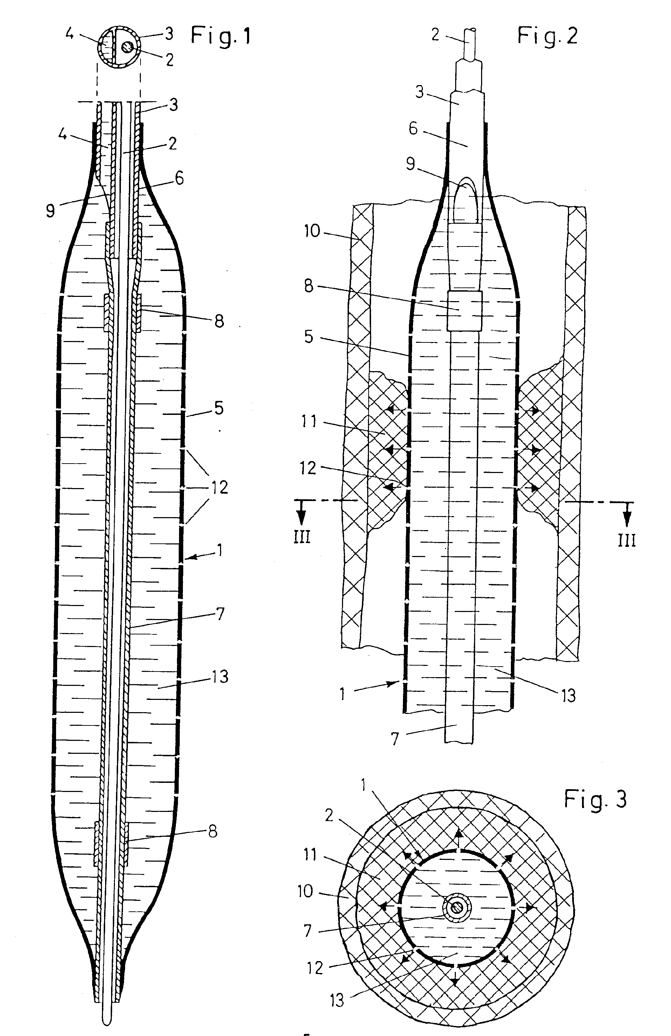

In den Fig. 2 und 3 ist mit 11 schematisch ein Gewebe gezeigt, das in einem Gefäss 10 eine Engstelle bildet. Im dilatierten Zustand liegt die Aussenseite der Wandung 5 am Gewebe 11 an und dringt Flüssigkeit 13 durch die Durchstiche 12 in das Gewebe 11. Die Flüssigkeit 13 wird somit direkt an das Gewebe 11 abgegeben.

Die Durchstiche 12 sind mit etwa gleichen Abständen auf der Wandung 5 verteilt, um eine entsprechend gleichmässige und verteilte Abgabe der Flüssigkeit 13 an das Gewebe 11 zu erreichen.

Die Flüssigkeit 13 enthält ein Behandlungs- und/oder Kontrastmittel. Im Falle der Behandlung einer Stenose ist das Behandlungsmittel insbesondere ein Mittel, welches das Gewebe 11 auflöst. Solche Mittel sind bekannt. Bei einer Behandlung wird das gefässverengende Gewebe 11 an die Gefässwand 10 gepresst und gleichzeitig unter Druck eine bestimmte Menge des Behandlungsmittels, in diesem Fall eine das Gewebe 11 auflösende Substanz, direkt in das Gewebe 11 eingebracht. Dadurch kann das Engstellengewebe 11 schneller und in mechanischer Hinsicht auch schonender entfernt werden.

**Patentansprüche**

1. Katheter, insbesondere zum Kanalisieren von Engstellen in Körperflüssigkeit führenden Gefässen, mit einem Führungskörper (2) und einem auf diesen aufzuschiebenden, an einem mehrlumigen Trägerschlauch (3) angeordneten Dilatationselement (1), das unter definitiver Ausdehnung mit einer durch den Trägerschlauch (3) eingebrachten Flüssigkeit (13) zu füllen ist, dadurch gekennzeichnet, dass das Dilatationselement (1) eine mikroporöse, für die Flüssigkeit permeable und an das Gewebe (11) der Engstelle anzulegen bestimmte Wandung (5) aufweist.

2. Katheter nach Anspruch 1, dadurch gekennzeichnet, dass das Dilatationselement (1) ein schlauchförmiger Ballon mit im ausgedehnten Zustand etwa zylindrischer Wandung (5) ist.

3. Katheter nach Anspruch 1 oder 2, dadurch gekennzeichnet, dass die Wandung (5) etwa radial diese durchdringende Poren (12) aufweist.

4. Katheter nach Anspruch 3, dadurch gekennzeichnet, dass die Poren (12) etwa gleichmässig über den Umfang und die Längserstreckung der Wandung (5) verteilt sind.

5. Katheter nach einem der Ansprüche 3 oder 4, dadurch gekennzeichnet, dass die Poren einen Durchmesser von etwa 0,05 mm aufweisen.

6. Katheter nach einem der Ansprüche 1 bis 5, dadurch gekennzeichnet, dass er zweilumig ist.

**Claims**

1. A catheter, more particularly for draining constrictions in vessels conveying body fluid, comprising a guide member (2) and an expansion element (1) which can be slid over the guide member and is disposed on a multiple-bore carrier tube (3) and can be expanded to a defined extent and filled with a fluid (13) introduced through the carrier tube (3), characterised in that the expansion element (1) has a wall (5) which is microporous, permeable to the fluid, and adapted to be placed against the tissue (11) at the constriction.

2. A catheter according to claim 1, characterised in that the expansion element (1) is a tubular balloon having a wall (5) which is approximately cylindrical when expanded.

3. A catheter according to claim 1 or 2, characterised in that the wall (5) has pores (12) which extend approximately radially through it.

4. A catheter according to claim 3, characterised in that the pores (12) are distributed approximately uniformly around the periphery and the length of the wall (5).

5. A catheter according to claim 3 or 4, characterised in that the pores have a diameter of about 0.05 mm.

6. A catheter according to claims 1 to 5, characterised in that it has two bores.

**Revendications**

1. Cathéter, notamment pour désobstruer des rétrécissements ou sténoses dans des vaisseaux conducteurs de fluide corporel, avec un corps de guidage (2) et un élément de dilatation (1) disposé sur celui-ci et à enfiler sur un tuyau flexible porteur (3) à plusieurs lumières, élément de dilatation (1) à remplir pour sa dilatation définitive d'un liquide (13) amené par le tuyau flexible porteur (3), cathéter caractérisé en ce que l'élément de dilatation présente une paroi (5) déterminée microporeuse perméable pour le liquide et destinée à être appliquée sur le tissu (11) de la sténose.

2. Cathéter selon la revendication 1, caractérisé en ce que l'élément de dilatation (1) est un ballonnet en forme de tuyau flexible comportant une paroi sensiblement cylindrique (5) à l'état dilaté.

3. Cathéter selon la revendication 1 ou 2, caractérisé en ce que la paroi (5) présente des piqûres ou pores (12) traversant celle-ci de façon sensiblement radiale.

4. Cathéter selon la revendication 3, caractérisé en ce que les piqûres ou pores (12) sont répartis de façon sensiblement uniforme sur la périphérie et sur l'étendue longitudinale de la paroi (5).

5. Cathéter selon l'une quelconque des revendications 3 ou 4, caractérisé en ce que les piqûres ou pores (12) présentent un diamètre d'environ 0,05 mm.

3

6. Cathéter selon l'une des revendications 1 à 6, caractérisé en ce qu'il comporte deux lumières.

Fig.1

Fig.2

Fig.3